Europäisches Patentamt

European Patent Office

Office européen des brevets

(19)

(11) Publication number: **0 107 305**
**A2**

# (12) EUROPEAN PATENT APPLICATION

(21) Application number: **83305191.5**

(22) Date of filing: **07.09.83**

(51) Int. Cl.³: **A 61 B 5/10**, G 01 B 5/24

(30) Priority: **30.09.82 US 431004**

(71) Applicant: **Sabia, Michael A., 81, Rumson Road, Little Silver New Jersey 07739 (US)**

(43) Date of publication of application: **02.05.84 Bulletin 84/18**

(72) Inventor: **Sabia, Michael A., 81, Rumson Road, Little Silver New Jersey 07739 (US)**

(74) Representative: **Kerr, Simonne June et al, European Patent Attorney POTTS, KERR & CO. 13 High Street, Windsor Berkshire SL4 1LD (GB)**

(84) Designated Contracting States: **CH DE GB IT LI**

(54) Scoliosis measuring apparatus.

(57) An apparatus for measuring the extent of scoliosis in an individual, a transverse support member (10), first (12) and second (14) mounting means predeterminedly positionable along the support member, a pair of extension members (20, 22) in individually co-operating relationship with each of the mounting means (12, 14) for resting at a selected body location of the individual when the apparatus is in use and adapted for removal from the mounting means (12, 14) when the apparatus is not in use, and means locatable on the support member for providing an angle indication (34) as to existing elevational differences between the extension members (20, 22) when rested at the body locations.

EP 0 107 305 A2

- 1 -

## SCOLIOSIS MEASURING APPARATUS

This invention relates to scoliosis, a curvature to areas of the spine in general, and to apparatus for providing a quantitative measure of the extent of any scoliosis present, in particular.

Two general methods exist by which the presence, or extent, of scoliosis in an individual can be determined. One, the least precise, is through visual observation of the alignment of a person's shoulders or hips when standing upright, and of his, or her, back when bending over. The other method commonly employs the use of x-rays, which, if measured, additionally provide information as to the extent of the scoliosis present.

Scoliosis is often treated by casting, manipulation, exercise programmes and operative techniques. Experience has shown, however, that the visual technique of observation does not adequately provide an indication as to whether progress is being made in reducing the curvature. While comparative x-rays — along with comparative measurements being made by the treating practitioner — provide accurate indications of any progress being made, such techniques are both time consuming, and costly (by virtue of the constant need for taking, developing and interpreting the x-ray), and also necessitates the availability of adequate x-ray equipment at the office location of the practitioner treating the patient.

It is to be appreciated that a fairly simple, inexpensive, quick and easy-to-use arrangement would be desirable to provide a quantitative analysis as to whether, and to what extent, the treatment and exercises accorded are producing the desired results in reducing the incipient curvature.

According to the present invention an apparatus for measuring the extent of scoliosis in an individual is characterized in that it comprises a transverse support member, first and second mounting means predeterminedly positionable along the support member, a pair of extension members in individually co-operating relationship with each of the mounting means for resting at a selected body location of the individual when the apparatus is in use and adapted for removal from the mounting means when apparatus is not in use and means locatable on the support member for providing an angle indication as to existing elevational differences between the extension members when rested at the body locations.

The angle indicator of appropriate configuration is arranged to sit atop the transverse rod, so as to provide a needle deflection, indicating which shoulder or hip -- and to what amount -- is higher than the other, in providing a quantitative determination as to the extent of scoliosis present. First and second positive locking arrangements are included, both to securely hold the extension rod in position for use with the patient and also in fixed position along the transverse rod at selected locations in accordance with the size and frame of the shoulder and hip positionings of the individual patient being tested.

In one embodiment of the invention, the extension rods are reticuled as an aid in affording a linear measurement to be made in determining the degree of scoliosis present in an area of the back, when the patient is bent over, with the transverse rod resting on his, or her, spine -- or, more generally, across the back area. Such reticulation also serves in measuring the progress of the scoliosis by releasing the rods to find the exact difference from one area of the posterior rib cage, deformable according to the severity of the rotation in the vertebral column.

The angle indicator, the extension rods and the slidable mounts on the transverse rod are all easily dismantable, so that the component parts making up the measuring apparatus can quickly be disassembled -- both for easy storage, or portable use. To such end, an angle indicator having a magnetic base proves particularly attractive in holding to a transverse rod, or member, affording a matching magnetic capability.

The invention will now be described further, by way of example, with reference to the accompanying drawings, in which:-

Fig. 1 is a perspective view of a scoliosis measuring device constructed in accordance with the invention

Fig. 2 is a cross-sectional view helpful in an understanding of the positive-locking aspects of the invention; and

Fig. 3 shows the measuring apparatus as it might appear for easy storage and/or carrying about.

In the drawings reference numeral 10 identifies the transverse support bar of the apparatus while reference numerals 12, 14 identify a pair of slidable mounts, positionably lockable in place by means of a threaded knob bolt 16 and washer 18 assembly.

A pair of extension rods 20, 22 each terminate in a knob end 24, and extend through the mounts 12, 14 to be respectively locked in place by threaded knob bolts 26, 28. A pair of apertures 30, 32 extend inwardly and through the mounts 12, 14, in co-linear alignment with the support bar 10. With the threaded knob bolts 16, 26 and 28 rotated to lock the extension rods 20, 22 in place, fixedly positioned along the transverse bar 10, the apparatus of the invention is ready for use in providing a quantitative indication of the extent of scoliosis present, once seated to rest across the shoulders and hips by means of the extension rods 20, 22 placed at such body location. (Measurements in the difference in the shoulder heights will also be able to be determined when the apparatus is placed in reverse position against the back and using the extension rods 20, 22 at the level of the shoulders to give a difference in millimeters, thereby affording the examiner both angular and millimeter evaluation.)

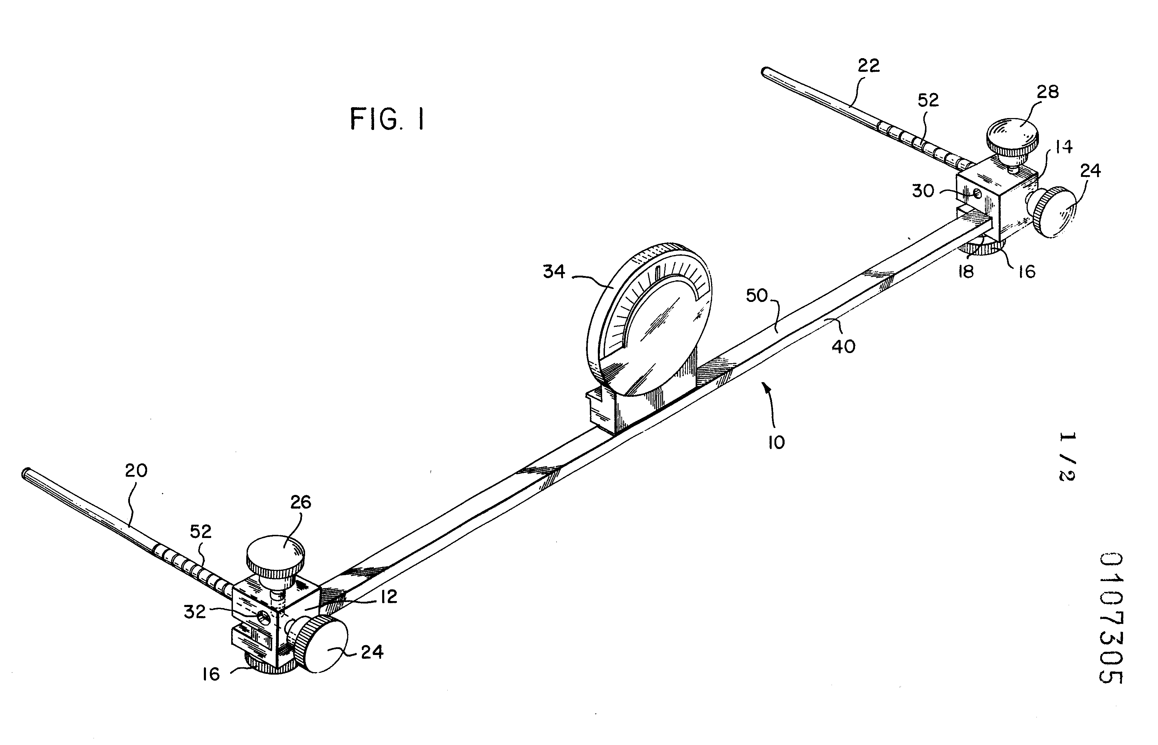

More specifically, there is shown in the drawing an appropriate angle indicating device 34, preferably of a magnetic base when the transverse support is constructed of a ferrous material. While it will be appreciated that any appropriate affixation method is sufficient, a magnetic coupling permits a very easy and quick, disassembly when the apparatus is not in use. Placement of the indicator 34 anywhere along the transverse support bar 10, as in Fig. 1, then provides an angle indication as to which shoulder (or hip) is higher, if either, and with angle gradations delineated in intervals of $1^{\circ}$, also provides a measure of the extent of elevation, indicative of the extent of scoliosis present. Although not to be construed as being so limited, applicant has found that an "angle finder" available from Sears-Roebuck & Co has proven useful in constructing one embodiment of the invention.

As will be apparent, the threaded knob bolts 16 permit the mounts 12, 14 to move in either direction, until the extension rods 20, 22 rest on the shoulders, or hips (i.e. in the area which is the acromio clavicular articulation), when the measurement is to be taken, depending upon the size and framing of the individual then being tested. By comparing the angular measurements at any given time, the treating practitioner can very simply determine whether the exercise and manipulation programme is being effective in achieving any of the control desired in reducing the scoliosis curvature. Instead of relying on mere visual observations to determine whether any positive effect is resulting — and instead of having to successively compare different x-ray pictures of the shoulders and hip views —, all that is necessary is for the practitioner to appropriately place the extension rods 20, 22, and read-off the indication of the angle measurer 34. By comparing the reading obtained — both in degrees and whether to the left, or right, of a "zero", vertical point, an effortless comparison, quantitative in nature, can be had by checking the reading obtained with one realized previously.

In similar manner, the measuring apparatus of the drawing can be used to determine quantitatively, the scoliosis at the patient's back location, merely by rotating the Fig. 1 apparatus 90° inwardly of the plane of the drawing, (so that the transverse bar 10 would lie across the back with the extension rods 20, 22 then pointed downwardly towards the floor area), and by repositioning and reorienting the angle indicator 34 so that it now sits atop the rod 10 on its surface 40, instead of on the support bar surface 50, as shown in Fig. 1. To afford the examinator a two phase check and an exacting measurement, the rods (which are calibrated in millimeters) are released, and at that point of level, find the exact difference from one area of the posterior rib cage that is deformed via severity of the rotation in the vertebral column. Again, previous angular and millimeter indications can be compared with that then obtainable, for a comparative interpretation as to the degree of success in the treatment and exercise programme being administered in an attempt to reduce the extent of exhibited scoliosis.

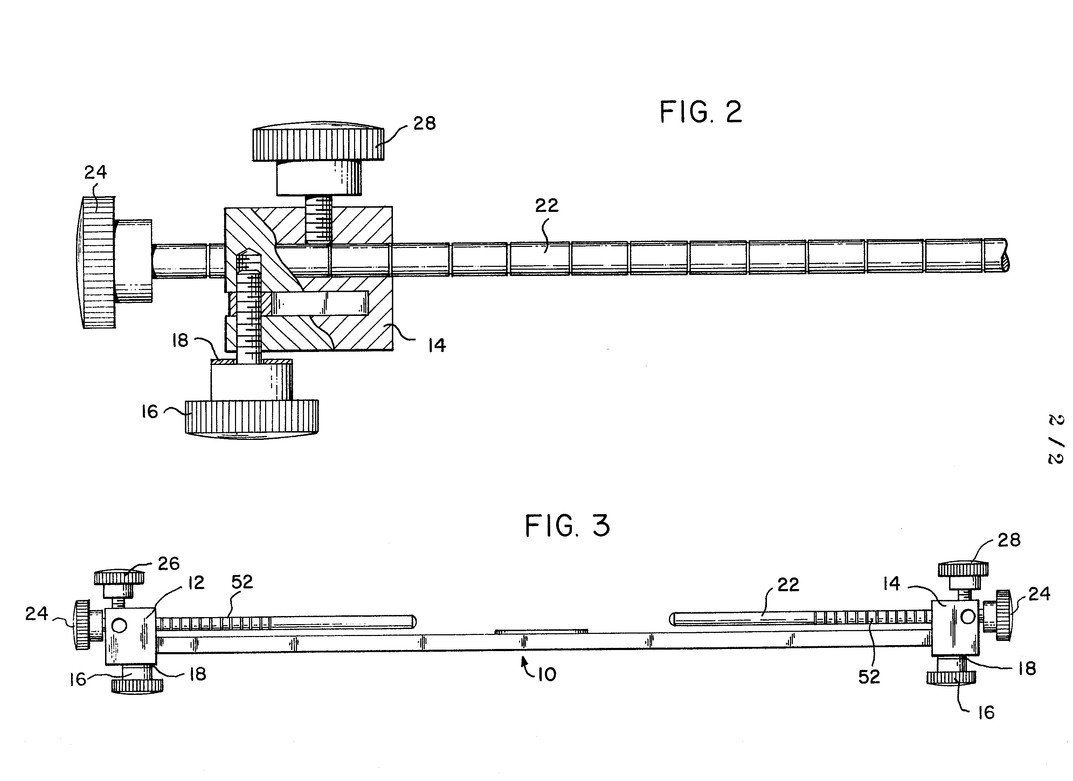

As will be readily appreciated, by incorporating an angle indicator 34 of a magnetic base, it can easily be removed for storage, when the unit is to be disassembled. As will also be apparent, by unlocking the knob bolts 16, the slidable mounts 12, 14 can be removed from the support bar 10, also for storage. In like manner, by unlocking the knob bolts 26, 28, the extension rods 20, 22 can be removed — with the result being that the entire measuring apparatus can be simply taken apart and stored, when not in use. As shown in Fig 3 an alternative arrangement can be had to completely dismantling the apparatus. There, - the angle indicator 34 continues to be removed, but with the slidable mounts 12, 14 being locked in place at the end terminations of the transverse support rod 10. The knob bolts 26, 28 are then loosened to permit removal of the extension rods 20, 22 , which are then re-inserted in the direction shown, through the co-linear apertures 30, 32 to lie substantially atop the transverse support rod 10 when not in use. Once so positioned, the bolts 26, 28 can, once again, be tightened to maintain the rods 20, 22 in such alignment, and the assembly can then be stored, or even packed to fit within a brief case, or attache case, and carried about. In one embodiment of the invention, the transverse support rod 10 was fabricated of steel of dimension approximating 1/2" x 1/4" x 18" — which, with the threaded knob bolts each being of a height less than one inch, made carrying about quite simple.

As more clearly shown in the drawing of Fig. 3, the extension rods 20, 22 are reticulated in delineations of some 3/16" each (reference numeral 52), so as to provide a further indicant — especially when used in measuring the back curvature with a patient bending at the waist. More specifically, once it is seen that an angle indication is produced by deflection in the unit 34, it becomes but a simple matter to raise the one extension rod 20, or 22, in a direction to bring the needle deflection back to zero in the indicator. With the reticulations on the rod, a linear measurement can be noted, and determined, as another quantitative indicant of the extent of scoliosis present at that time. Subsequent comparisons made at a later date, after treatment, exercise programmes, etc. have transpired, is then effective as showing a measure of success through the treatment accorded.

0107305

## CLAIMS

1. An apparatus for measuring the extent of scoliosis in an individual characterized in that it comprises a transverse support member (10), first (12) and second (14) mounting means predeterminedly positionable along the support member, a pair of extension members (20,22) in individually co-operating relationship with each of the mounting means (12, 14) for resting at a selected body location of the individual when the apparatus is in use and adapted for removal from the mounting means (12,14) when the apparatus is not in use, and means locatable on the support member for providing an angle indication (34) as to existing elevational differences between the extension members (20,22) when rested at the body locations.

2. An apparatus according to claim 1 characterized in that the angle indication means (34) is adapted for removal from the support member (10) when the apparatus is not in use.

3. An apparatus according to claim 1, characterized in that the angle indication means (34) is locatable on the support member (10) in magnetically attracting relationship.

4. An apparatus according to claim 1, characterized in that adjustable locking means (16, 18) are provided for holding the mounting means (12,14) in place along the support member (10) when the apparatus is in use and for permitting removal of the mounting means (12,14) from the support member (10) when the apparatus is not in use.

5. An apparatus according to claim 1, characterized in that adjustable locking means (26,28) are provided for holding the extension members (20,22) in co-operating relationship with the mounting means (12,14) when apparatus is in use and for permitting removal of the extension members (20,22) from the mounting means (12,14) when the apparatus is not in use.

6. An apparatus according to claim 1, characterized in that the mounting means (12,14) are apertured (30,32) to align the extension members (20,22) in a plane perpendicular to the longitudinal axis of the transverse support member (10) when the apparatus is in use.

7. An apparatus according to claim 6, characterized in that the mounting means (12,14) are also apertured (30,32) to align the extension members (20,22) in a plane co-linear with the longitudinal axis of the transverse support member (10) when the apparatus is not in use.

- 7 -

0107305

8.   An apparatus according to claim 1, characterized in that the extension members (20,22) are reticulated along their length in predetermined amounts, for providing a linear indication as to existing elevational differences between the extension members (20,22) when rested at selected body locations of the individual.

FIG. I

0107305

0107305

FIG. 2

FIG. 3